# EUROPEAN PATENT APPLICATION

(11) **EP 2 601 893 A1**
(43) Date of publication of application: **12.06.2013**
(21) Application number: 12196067.8
(22) Date of filing: 07.12.2012
(51) Int. Cl.: A61B 8/00

(54) **Ultrasonic diagnostic probe and apparatus including the same**

(30) Priority: 08.12.2011 KR 20110130792
(71) Applicant: Samsung Medison Co., Ltd., Gangwon-do 250-870 (KR)
(72) Inventor: Lee, Jun Yeol, Daegu (KR)
(74) Representative: Schmid, Wolfgang

(57) **Abstract**

An ultrasound diagnostic apparatus having a probe configured to transmit and receive an ultrasonic wave. The diagnostic probe includes a driving unit. A transducer is configured to rotate by receiving a rotating force generated by the driving unit A rotary shaft of the driving unit is directly connected to the transducer, and thus the components configured for the delivery of a driving force are cancelled, and thereby the composition of the probe is simplified.

## Description

### Technical Field

The present disclosure relates to an ultrasonic diagnostic apparatus having a probe configured to transmit an ultrasonic wave at a subject to be diagnosed, and to receive the ultrasonic wave that is reflected back from the subject.

### Background

In general, an ultrasonic diagnostic apparatus is an apparatus configured to radiate an ultrasonic wave toward a portion to be diagnosed at an inside a body from a body surface of a subject to be diagnosed, and to obtain an image of a cross section of a soft tissue or a blood flow through the ultrasonic wave that is reflected.

The ultrasonic diagnostic apparatus as such includes a body; a probe configured to transmit an ultrasound wave signal to a subject, and to receive the signal that is reflected from the subject; a display unit disposed at an upper side of the body, and configured to display a diagnostic result obtained through the received ultrasonic wave as an image; and a control panel disposed at a front side of the display unit for a user to control the ultrasonic diagnostic apparatus.

The probe from the above component includes a transducer to transmit and receive an ultrasonic wave, and in recent years, an ultrasonic diagnostic apparatus, which is configured to obtain a three-dimensional image by rotating the transducer, is provided.

### Summary

Therefore, it is one aspect of the present disclosure to provide an ultrasonic diagnostic apparatus configured to rotate a transducer of a probe of the ultrasonic diagnostic apparatus in a further simplified manner.

In accordance with one aspect of the present disclosure, an ultrasound diagnostic apparatus includes a probe configured to transmit and receive an ultrasonic wave. The probe includes a driving unit configured to generate a rotational force. A transducer is configured to rotate by receiving the rotational force generated by the driving unit. A rotary shaft of the driving unit is directly connected to a center of rotation of the transducer.

The transducer may have a radius of gyration smaller than a radius of curvature of the transducer.

The ultrasound diagnostic apparatus may further include a handle case and a cap. The handle case may be configured to be grasped by a user. The cap may be disposed at a front end of the handle case and provided with the driving unit and the transducer accommodated therein.

The handle case may be provided at both sides of the front end thereof with a pair of hinge units on which both sides of the transducer are rotatively installed, respectively.

The transducer may include a pair of hinge protrusions rotatively installed at the pair of hinge units while being protruded toward side directions from the both sides thereof. The pair of hinge units may include hinge holes at which the hinge protrusions are installed.

The driving unit may be disposed at an inner side of the transducer, and the rotary shaft of the driving unit may be directly connected to an inner side of a portion at which the hinge protrusion of the transducer is formed.

The driving unit may include a stepping motor.

The driving unit may include a geared motor that is integrally formed with a deceleration unit to perform deceleration.

As described above, the probe applied at the ultrasonic diagnostic apparatus in accordance with the present disclosure is provided with the rotary shaft of the driving unit thereof directly connected to the center of the rotation of the transducer, and thus the components configured for the delivery of driving force may be omitted, and thereby the configuration of the probe is simplified.

In addition, as described above, since the driving unit is installed at an inside the cap, the shape of the handle case may be designed in various shapes so that a user can easily grasp the handle case.

In addition, as described above, since the radius of gyration of the transducer is provided to be smaller than the radius of curvature of the transducer, the sizes of the transducer and the cap are reduced.

In another aspect, a diagnostic probe for an ultrasonic diagnostic apparatus configured to transmit and receive an ultrasonic wave is provided. The probe comprises a user handle. A driving unit is disposed adjacent to the user handle. The driving unit is configured to generate a rotational force. A transducer is configured to rotate by receiving the rotational force generated by the driving unit. A rotary shaft of the driving unit is directly connected to a center of rotation of the transducer.

Additional advantages and novel features will be set forth in part in the description which follows, and in part will become apparent to those skilled in the art upon examination of the following and the accompanying drawings or may be learned by production or operation of the examples. The advantages of the present teachings may be realized and attained by practice or use of various aspects of the methodologies, instrumentalities and combinations set forth in the detailed examples discussed below.

### Brief Description of the Drawings

These and/or other aspects of the disclosure will become apparent and more readily appreciated from the following description of the examples, taken in conjunction with the accompanying drawings of which:
FIG. 1 is a perspective view of an ultrasonic diagnostic apparatus in accordance with one example of the present disclosure.
FIG. 2 is an exploded perspective view of a probe applied to the ultrasonic diagnostic apparatus in accordance with an example of the present disclosure.
FIG. 3 is a partial cross-sectional view of the probe applied to the ultrasonic diagnostic apparatus in accordance with an example of the present disclosure.
FIG. 4 is a partial cross-sectional view of a probe applied to an ultrasonic diagnostic apparatus in accordance with another example of the present disclosure.
FIG. 5 is a partial cross-sectional view of a probe applied to an ultrasonic diagnostic apparatus in accordance with still yet another example of the present disclosure.

### Detailed Description

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and circuitry have been described at a relatively high-level, without detail, in order to avoid unnecessary obscuring aspects of the present teachings.

Reference will now be made in detail to examples of the present disclosure, which are illustrated in the accompanying drawings, wherein like reference numerals refer to like elements throughout.

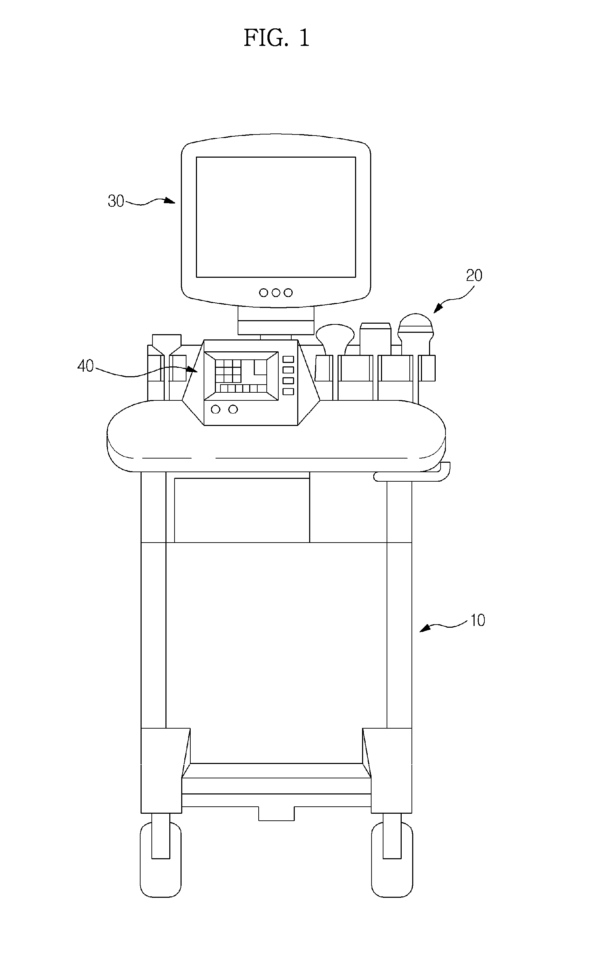

As illustrated in the example of FIG. 1, an ultrasonic diagnostic apparatus includes a body 10, a probe 20 to transmit an ultrasonic signal to a subject to be diagnosed and to receive the reflected signal from the subject, a display unit 30 disposed at an upper side of the body 10 and configured to display a diagnostic result obtained from the received ultrasonic signal as an image, and a control panel 40 configured in a way for a user to manipulate the ultrasonic diagnostic apparatus.

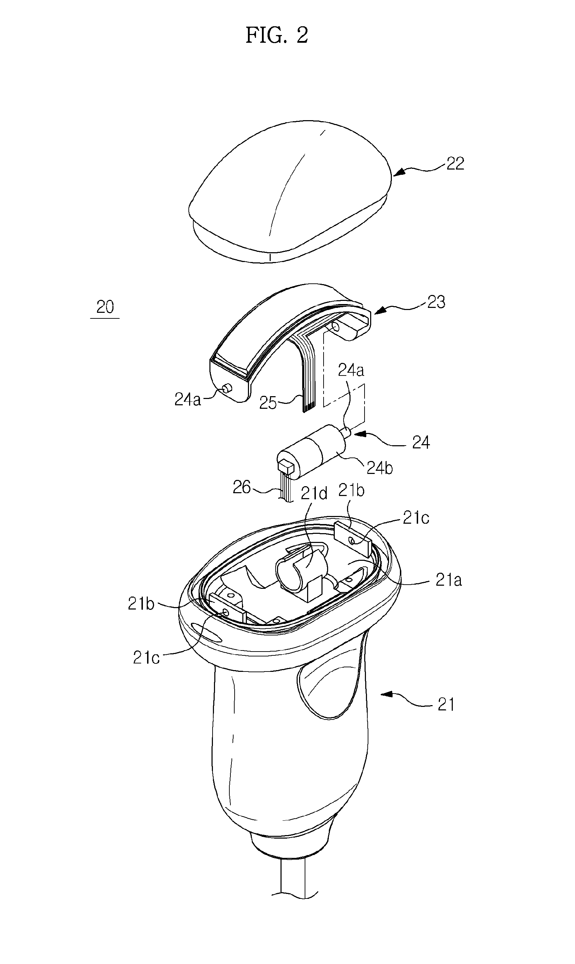

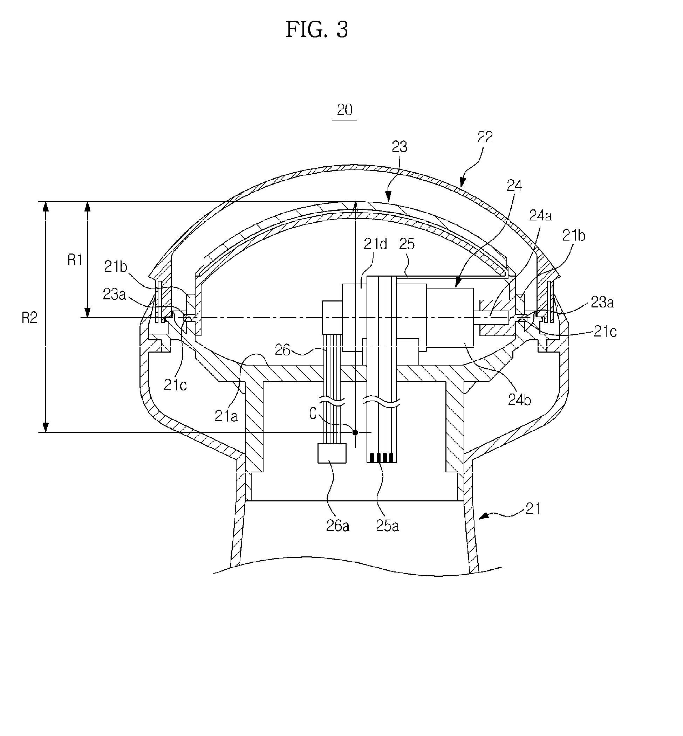

As illustrated on FIGS. 2 to 3, the probe 20 includes a handle case 21 configured to be grasped by a user, and a cap 22 disposed at a front end of the handle case 21 to be in contact with the subject to be diagnosed, a driving unit 24 to generate rotational force, and a transducer 23 to rotate by receiving the rotational force from the driving unit 24.

The driving unit 24 includes a rotary shaft 24a that is connected to the transducer 23 and configured to deliver the transducer 23 with a rotational force by rotating forward and backward. The driving unit 24 is implemented with a driving motor configured to generate a rotational force by receiving power, for example, a stepping motor capable of precisely controlling a rotating angle of the rotary shaft 24a. In addition, the driving unit 24 is implemented with a geared motor that is provided with a deceleration unit 24b integrally formed at one side of the driving unit 24 so as to decelerate the driving unit 24.

The transducer 23 includes an ultrasonic wave transducer configured to transmit and receive an ultrasonic wave, and as described above, is capable of reading a three-dimensional image of a subject to be diagnosed while rotatively installed at an inside the cap 22.

A portion of the cap 22 corresponding to the transducer 23 is provided with a cross section having an arc shape, so that, even when the transducer 23 installed at an inside the cap 22 is rotated, the gap between an inner surface of the cap 22 and an outer surface of the transducer 23 may be maintained in a constant manner. The space in between a front end surface 21a of the handle case 21 and the cap 22 is filled with oil configured to deliver an ultrasound wave.

The handle case 21, as described above, is provided at the front end thereof with the cap 22 installed thereto, and is provided at both sides of the front end surface 21a thereof with a pair of hinge units 21b, which is protruded toward an inner side of the cap 22 so that both sides of the transducer 23 are rotatively installed thereto.

The both sides of the transducer 23, through the pair of hinge units 21b described above, are rotatively installed at an inside the cap 22. For such, at the both sides of the transducer 23, a pair of hinge protrusions 23a protruded toward both side directions are formed, and the two hinge units 21b have hinge holes 21c at which the two hinge protrusions 23a are rotatablely installed, respectively. In the example, a radius of gyration R1 of the transducer 23 is smaller than a radius of curvature R2 of the transducer 23, that is, smaller than the distance from the center of curvature C of the transducer 23 to the outer circumferential surface of the transducer 23. By composing the radius R1 of gyration of the transducer 23 as such, the sizes of the transducer 23 and the cap 22 are reduced. As the size of the transducer 23 is reduced, the inertial moment acting on the transducer 23 is reduced, so that the rpm of the transducer 23 is increased while using the driving unit 24 having the same performance. In addition, as the size of the cap 22 is reduced, the amount of medium to be filled in the cap 22 is reduced, advantageously resulting in a slimness of the probe 20.

In addition, the driving unit 24 described above is disposed at an inner side of the cap 22 while being installed at the front end surface 21a of the handle case 21, and the rotary shaft 24a of the driving unit 24 is directly connected to the center of the rotation of the transducer 23. That is, the rotary shaft 24a is directly connected to an inner side of a portion at which the hinge protrusion 23a of the transducer 23 is formed. At the front end surface 21a of the handle case 21, a motor bracket 21d configured for the installation of the driving unit 24 is provided.

As described above, when the driving unit 24 is disposed at an inner side of the cap 22 and when the rotary shaft 24a of the driving unit 24 is directly connected to the center of the rotation of the transducer 23, the components configured for the delivery of the driving force may be canceled, and thus the composition of the probe 20 is further simplified.

In addition, the probe 20 includes a flexible printed circuit board 25 configured to deliver power and signals to the transducer 23, and a cable 26 to supply power to the driving unit 24.

The flexible printed circuit board 25 is provided with one end thereof connected to the transducer 23, while the other end thereof is extended to an inside of the handle case 21 after penetrating through the front end surface 21a of the handle case 21. At the other end of the flexible printed circuit board 25, a first connector unit 25a is provided for a connection.

The cable 26 is provided with one end thereof connected to the driving unit 24, and the other end thereof is extended to an inside of the handle case 21 after penetrating through the front end surface 21a of the handle case 21. At the other end of the cable 26, a second connector unit 26a is provided for a connection.

As described above, at an inner side of the cap 22, the driving unit 24 is disposed, and at an inside the handle case 21, only the first connector unit 25a and the second connector unit 26a are disposed. The first connector unit 25a and the second connector unit 26a are the components occupying a small space, and if needed, the first connector unit 25a and the second connector unit 26a may be formed in a significantly reduced size. Thus, a designer of the probe 20 may be able to design the shape of the handle case 21 in various shapes so that a user can easily grasp the handle case 21.

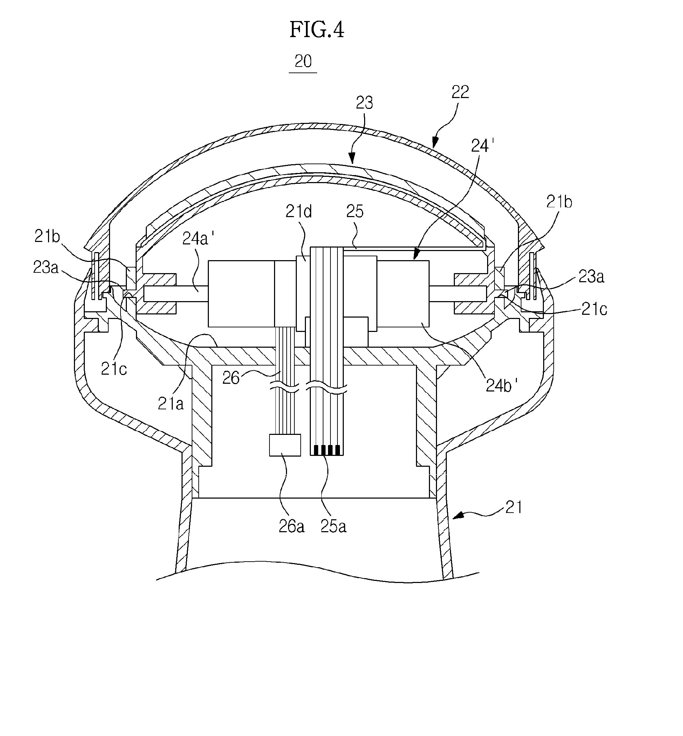

Although the rotary shaft 24a of the driving unit 24 in accordance with the embodiment has only one end being connected to the transducer 23, the present disclosure is not limited thereto. As shown in FIG. 4, a rotary shaft 24a' of a driving unit 24' may have opposite ends being connected to a transducer 23'.

Although the driving unit 24 in accordance with the embodiment is implemented with a geared motor, the present disclosure is not limited thereto. The driving unit 24 may be implemented with a general driving motor.

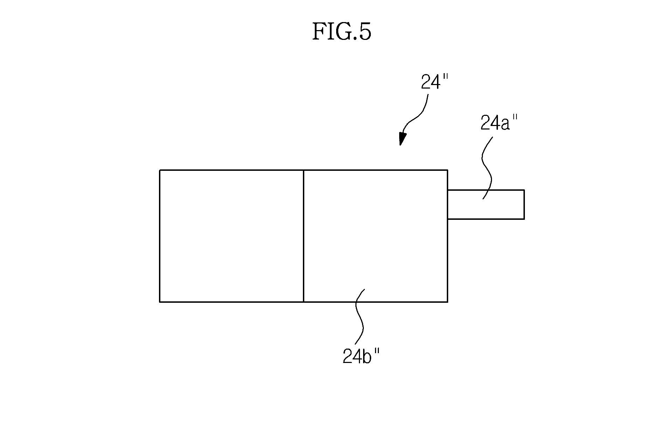

Although the rotary shaft 24a of the driving unit 24 is disposed at the center of the driving unit 24, the present disclosure is not limited thereto. As shown in FIG. 5, in the case of a driving unit 24" implemented with a geared motor having a deceleration unit 24b" formed at one side thereof, a rotary shaft 24a" may be disposed at a side away from the center of the driving unit 24" depending on the design of gears included at an inside the driving unit 24".

While the foregoing has described what are considered to be the best mode and/or other examples, it is understood that various modifications may be made therein and that the subject matter disclosed herein may be implemented in various forms and examples, and that the teachings may be applied in numerous applications, only some of which have been described herein. It is intended by the following claims to claim any and all applications, modifications and variations that fall within the true scope of the present teachings.

## Claims

1. An ultrasound diagnostic apparatus, comprising:
a probe configured to transmit and receive an ultrasonic wave,
wherein the probe comprises:
a driving unit configured to generate a rotational force; and a transducer configured to rotate by receiving the rotational force generated by the driving unit, and
a rotary shaft of the driving unit is directly connected to a center of rotation of the transducer.

2. The ultrasound diagnostic apparatus of claim 1, wherein:
the transducer has a radius of gyration smaller than a radius of curvature of the transducer.

3. The ultrasound diagnostic apparatus of claim 1, further comprising:
a handle case configured to be grasped by a user; and
a cap disposed at a front end of the handle case and provided with the driving unit and the transducer accommodated therein.

4. The ultrasound diagnostic apparatus of claim 3, wherein:
the handle case is provided at both sides of the front end thereof with a pair of hinge units on which both sides of the transducer are rotatively installed, respectively.

5. The ultrasound diagnostic apparatus of claim 4, wherein:
the transducer comprises a pair of hinge protrusions rotatively installed at the pair of hinge units while being protruded toward side directions from the both sides thereof, and
the pair of hinge units comprises hinge holes at which the hinge protrusions are installed.

6. The ultrasound diagnostic apparatus of claim 5, wherein:
the driving unit is disposed at an inner side of the transducer, and the rotary shaft of the driving unit is directly connected to an inner side of a portion at which the hinge protrusion of the transducer is formed.

7. The ultrasound diagnostic apparatus of claim 1, wherein:
the driving unit comprises a stepping motor.

8. The ultrasound diagnostic apparatus of claim 1, wherein:
the driving unit comprises a geared motor that is integrally formed with a deceleration unit to perform deceleration.

9. The ultrasound diagnostic apparatus of claim 8, wherein:
the rotary shaft is disposed at a side away from a center of the driving unit.

10. The ultrasound diagnostic apparatus of claim 1, wherein:
the rotary shaft has opposite ends being connected to the transducer.
